# EUROPEAN PATENT SPECIFICATION

(11) **EP 3 897 473 B1**
(45) Date of publication and mention of the grant of the patent: **14.08.2024**
(21) Application number: 19821150.0
(22) Date of filing: 15.11.2019
(51) Int. Cl.: A61F 2/97, A61F 2/954

(54) **STENT DEVICE DELIVERY TOOL**
FREISETZUNGSINSTRUMENT FÜR EINE STENTVORRICHTUNG
INSTRUMENT DE POSE DE DISPOSITIF D'ENDOPROTHÈSE

(30) Priority: 20.12.2018 GB 201820899
(43) Date of publication of application: 27.10.2021
(73) Proprietor: Vascutek Limited, Renfrewshire PA4 9RR (GB)
(72) Inventor: MCDONALD, Gary, Glasgow Strathclyde G62 7JZ (GB)
(74) Representative: Murgitroyd & Company
(86) International application number: PCT/GB2019/053238
(87) International publication number: WO 2020/128417

(56) References cited:
- WO-A1-2016/054537
- US-A1- 2006 015 171
- US-A1- 2006 184 226

## Description

The present invention relates to a stent device delivery tool.

In this connection, current treatment methods for aortic dissections and aneurysms predominantly utilize conventional surgical grafts and open surgery. Although endovascular treatment methods are also possible, the complexity of maintaining profusion to all major branch vessels radiating from the top of the aortic arch by purely an endovascular approach, means that endovascular treatment methods are currently very limited.

Furthermore, the use of conventional surgical grafts often necessitates a full thoracotomy, namely a major surgical opening of the chest cavity, which typically necessitates a Coronary Artery Bypass and the need to induce hypothermia and cardiac arrest. Undertaking such surgical procedures is not without risk of further complications.

Moreover, known endovascular stent device delivery systems rely on an internal delivery support shaft, typically having an integral moulded tip at its end to facilitate insertion, as well as mounting loops and release wire to support and deploy the device. This type of arrangement allows the device to be suspended from the delivery system at the tip end. It is held there until unsheathing has been performed, after which it can then be released from the delivery system. After release, which is typically by the removal of a release wire, the supporting internal central shaft and tip assembly must then be fully removed from within the stent device by retracting these items through the inside of the device lumen. These items must be removed carefully to overcome the potential risk of dislodging the previously deployed device by unintentional snagging. To provide this functionality, the delivery system typically necessitates other adjunctive elements such as a guide-wire, which would pass through the internal lumen of the shaft and tip moulding.

Such components prohibit anastomosing the non-stented end of the device to either an adjunctive device or native vessel prior to deployment of the stented section, should that be required.

US2006/184226 discloses a medical appliance including an outer sheath enclosing a self-expanding stent.

An object of the present invention is to provide an improved stent delivery system that can alleviate problems associated with what is currently available.

According to the present invention there is provided stent device as defined by claim 1.

Such an apparatus simplifies the stent device delivery system in terms of its complexity, which together with the reduction in components provides the user with fewer procedural steps and potential risks, enabling a more time efficient and simplified device deployment. It moreover allows the potentially complex stent device deployment procedure to be controlled by a single user.

Furthermore, as the apparatus has no central shaft or support that projects into the vessel or lumen, a greater degree of freedom is afforded during the procedure in terms of connection options and timings. The apparatus furthermore presents the stent device as a relatively slim, stiff column with a small diameter, meaning that it can deployed in narrower vessels.

The bore may preferably comprise one or more apertures and these may take the form of two circumferential slots extending in the longitudinal direction of the bore. Alternatively, the bore restriction may take the form of one or more pins or prongs that engage with the stent device.

Conveniently, the slots subtend an angle in the range of 90 to 120 degrees circumferentially about the centre of the bore.

Conveniently, the apparatus further comprises an end collar at the distal end of the body. The inward facing walls of the body may be arranged to taper inwardly towards the end collar thereby guiding the sheathing material to the collar.

Conveniently, the body further comprises a side window allowing a section of the stent device to branch off the longitudinal axis of the sheathed section with the sheath still mounted in the body.

In this connection, the side window preferably provides a pathway, substantially perpendicular to the axis of the bore, for the stent device to pass out from the apparatus body and sheath material.

The body comprises an openable cover for allowing access into its interior. In this way, loading and unloading of the stent device into apparatus can be facilitated.

The openable cover is hingeably attached.

The cover is lockable in a closed position by way of a locking pin.

The locking pin is couplable to a sheath material pull strap, the pull strap further being couplable to sheath material at the distal end of the body. Preferably, the locking pin is activated to be removed from the body to unlock the cover, only once a predetermined extent of sheath material has been pulled from the distal end of the body.

The locking pin may be coupled to the pull strap by a cord that has a controlled extent of slack relative to the extent of sheath material coupled to the pull strap.

Preferably, the apparatus further comprises a handle coupled to the body, the handle having an aperture through which the sheath material at the distal end of the body extends to the pull strap.

Conveniently, the handle is coupled to the body by way of one or more flexible members.

Conveniently, the bore has a profiled element, for example a blunt wedge element for promoting splitting of a stent device sheath within the bore.

Preferably, the wedge element presents a rounded surface to the sheath as it advances towards the distal end of the body.

According to a further aspect of the disclosure there is provided a method for deploying a stent device using apparatus as claimed in any preceding claim, the method comprising the steps:- inserting an at least partially sheathed stent device within said body bore with a portion of the sheathed stent device projecting from a proximal end of the body and a distal end of the stent device abutting said restriction within the bore, wherein sheath material at the distal end of the stent device extends past said bore restriction for access at a distal end of the body; presenting the proximal end of the sheathed stent device within a target vessel; and pulling said sheath material from the distal end of the body to urge the stent device against the bore restriction, thereby allowing the sheath material to be pulled back relative to the stent device to expose the proximal end of the stent device and be deployed within the vessel.

By externalising the delivery system around the stented prosthesis, there is not the need to have a delivery shaft, mounting loops and release wire to support and deploy the device.

Furthermore, as the externalised delivery system has no central shaft or support passing through the device lumen, this enables the option of anastomosing the non-stented end of the device to either an adjunctive device (or native vessel) prior to deployment of the stented section, if that is required. In terms of connection options and timings, this increases the scope to perform the procedure.

Embodiments will now be describe by way of example and with reference to the following drawings, of which:-
Figure 1 shows a cross-sectional view of the apparatus of the present invention with a compatible stent device in place;
Figures 2a to 6d show the stages of operation of embodiments of the present invention in deploying a compatible stent device;
Figures 7a and 7b show views of a compatible stent device for use with the present invention; and

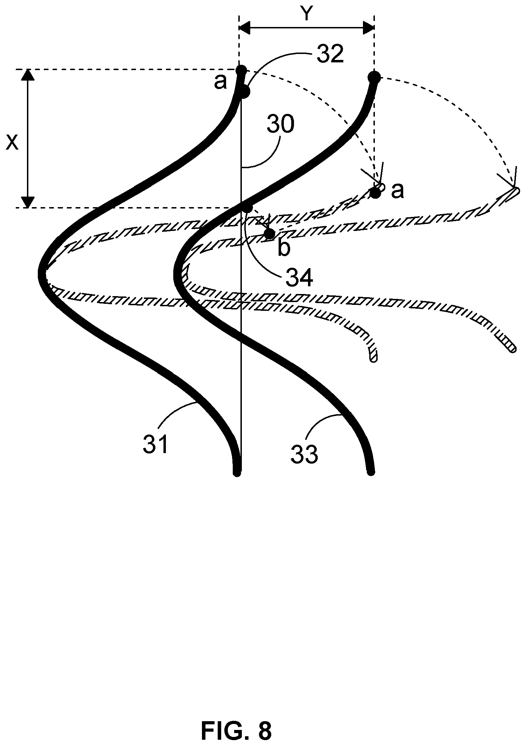

Figure 8 is a schematic view showing adjacent ring elements of a compatible stent device for use with the present invention.

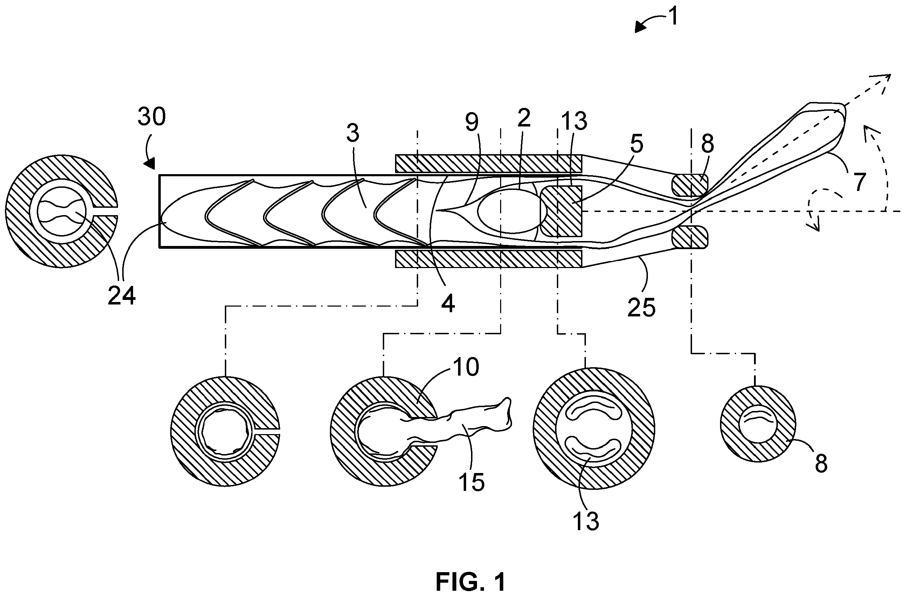

Figure 1 shows a cross-sectional view of the apparatus 1, having a body 2 into which a compatible sheathed stent device 3 is located.

In this regard, the body has a bore 4, dimensioned to allow the sheathed stent device 3 to sit within the bore, but not so tight so as to prevent the sheath material from moving relative to the bore and the stent device.

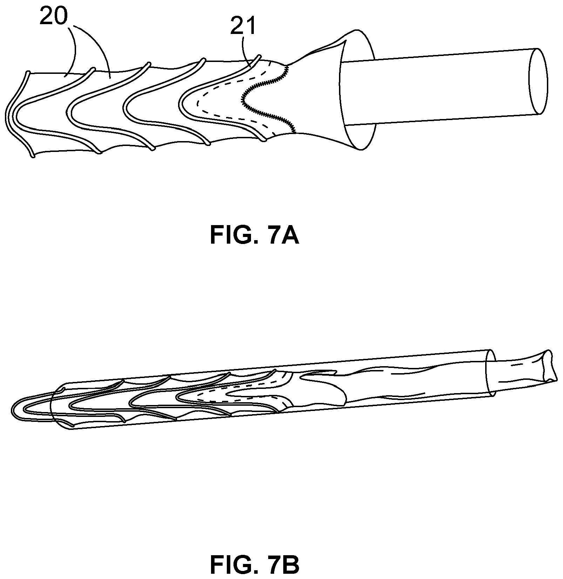

The compatible sheathed stent device 3 for use with the apparatus is shown in Figures 7a and 7b. This preferably comprises a lumen or sleeve of fabric, typically gel coated polyester, fitted with a series of spring like "stent elements" 20, typically having ring elements 21 formed from nitinol wire in the shape of an undulating Z or in the preferred embodiment a saddle ring, namely a hyperbolic paraboloid.

Multiple ring elements 21 of the compatible sheathed stent device are located along the axis of the lumen and these are attached circumferentially to the fabric by sutured thread, to form the stented device section, which has the capacity to be constrained into a significantly smaller diameter tube, namely the sheath.

When compacted into a small calibre sheath as shown in Figure 7b, the compatible sheathed stent device (with an appropriate selected oversize) can be readily inserted into the lumen of a branch vessel. Removing the sheath from the stent device enables it to be deployed into the native vessel, where the stented section expands radially outwards. The radial expanding stent elements contact and push onto the internal vessel wall to create a snug fitting non-sutured sealed junction.

The overlapping nature of the ring elements in the compacted configuration affords the sleeve with a column stiffness facilitating use with deployment apparatus.

In this example of the compatible device, the sheath may be configured with a relative high saddle height and a ring inter spacing which is less than the saddle height, to provide an overlap of the peaks and valleys of adjacent rings. This property in combination with an adjacent section of supported fabric is utilised to maintain the position of the stent device relative to the body 2, prior to and during the unsheathing process.

As shown in Figure 1, at or near the distal end of the stented section of the compatible device, there may be provided a section of flexible crimped fabric 15, typically gel coated polyester, which is joined and attached by suturing to form a blood tight continuous endoprothesis lumen. In some embodiments this section may also include a "Y" branch lumen. The non-stented section is provided to enable an endoprothesis to be joined by suturing to either the main prosthesis body or alternatively to a healthy section of native vessel, to reinstate blood profusion to the native branch vessel.

The sheath of the compatible stent device is preferably thin walled (typically a PTFE material), which has an inherent preposition to tear linearly, without the need for additional grooves or perforations. The sheath may have three sections: a proximal circular section, which has a length slightly longer than the length of the compacted stented section, a tail section at its distal end and a mid-section, where the circular section splits and propagates into the two tail elements.

These flat ribbon like tail elements 7 originate from the end of the circular section and can be formed by folding. The formed tails are fed through or past a restriction 5 within the body 2 and out into a separate strap element, where they can be tied together to form a singular user interface for sheath removal.

As shown in Figure 1, the restriction 5 in the bore 2 is configured to obstruct travel of the stent device. The restriction however allows stent device sheath material, namely the tails 7 to pass the restriction for access at the distal end of the body 2. The sheath material exits the body by way of a collar 8, whose core aperture has rounded exit surfaces to allow the sheath material to be pulled from a wide range of angles on exiting the body 2.

Whilst any suitable means may be employed to allow passage of the sheath material past the restriction, in the embodiment shown in Figure 1 two arcuate apertures 13 are provided in the face of the restriction 5, the apertures extending longitudinally in the axial direction of the body. The apertures are substantially circumferential and subtend an angle of 90 to 120 degrees. In this connection, the apertures each provide passage for a tail of sheath material 7, the sheath material being split within the bore 4 at point 9.

The body has a side window 10 for allowing the sheathed stent device to be positioned within the body with a crimped section 15 of the stent device exiting the body to the side via the window. The side window hence provides a pathway for the non-stented device fabric to pass through out from the confines of body 2 substantially perpendicularly to the axis of the sheathed sheath, enabling access to the distal end of the stent device. This end can hence be trimmed in length to suit individual patient anatomy and facilitates suturing to an adjunctive graft or native vessel.

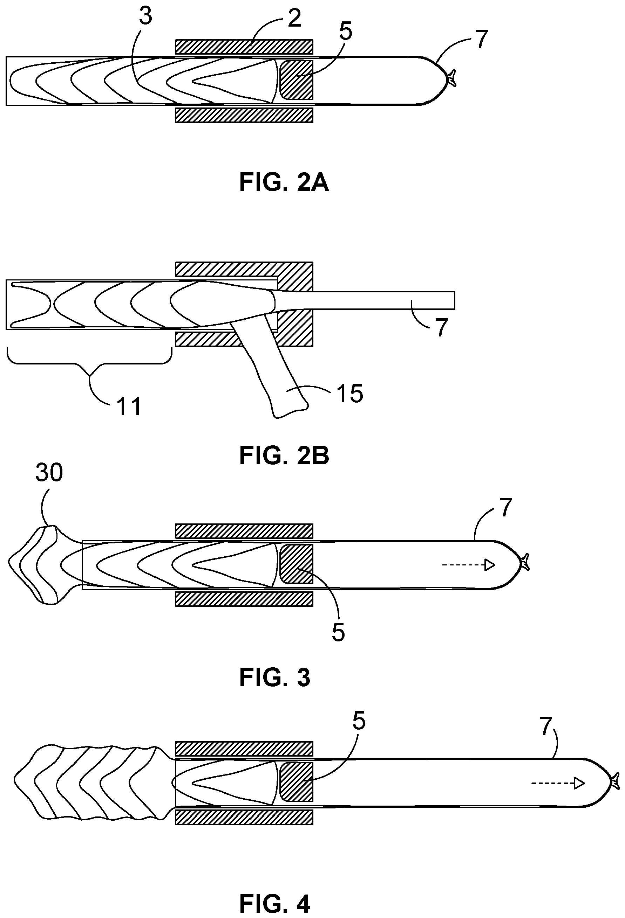

A sequence of use of embodiments of the apparatus of the present invention is shown in Figures 2a to 6d.

As shown in Figures 2a and 2b, the compatible sheathed stent device 3 is inserted into the body of the apparatus with tails 7 of the sheath projecting from the distal end, and a sheathed stent device section 11 projecting from the proximal end of the body. The sheathed section 11 is relatively rigid, by virtue of the interaction of the compressed stent device material, wishing to expand against the inside surface of the sheathing material. As such it can be readily and accurately manipulated and moved into position within a vessel or lumen requiring the stent procedure.

In this regard, the compatible stent device is preferably formed of imbricated ring elements, which are configured and arranged on a fabric lumen, so when compacted and constrained within the sheath material 7, they contribute to provide an element, which exhibits a high degree of column stiffness.

Once in position within the lumen or vessel, pulling the sheath material tails 7 draws the stent device into engagement with the restriction 5 so that the stent device itself cannot move further. In so doing, further pulling of the sheathing material results in the sheathing material being pulled back from the proximal end 30 of the stent device so that the stent device becomes exposed at its proximal end and hence can expand towards the deployed state within the lumen or vessel. This is illustrated in Figure 3. Figure 4 shows the tails 7 being pulled yet further to expose more of the stent device so that more of it can deploy within the lumen or vessel.

As shown in Figure 1, the distal end 25 of the body 2 is tapered to guide the sheathing material towards the collar 8. In this connection, exit surfaces of the collar 8 are rounded to allow the tails to be pulled over a wide range of angles without tearing or fracturing.

Once the stent device has been sufficiently deployed, it can be removed from the body 2 of the apparatus.

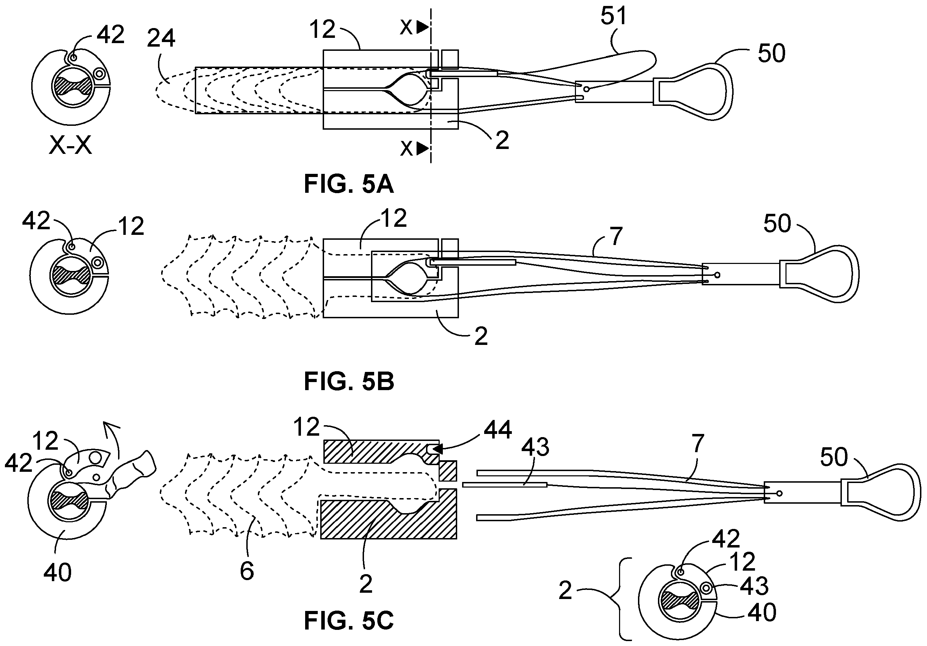

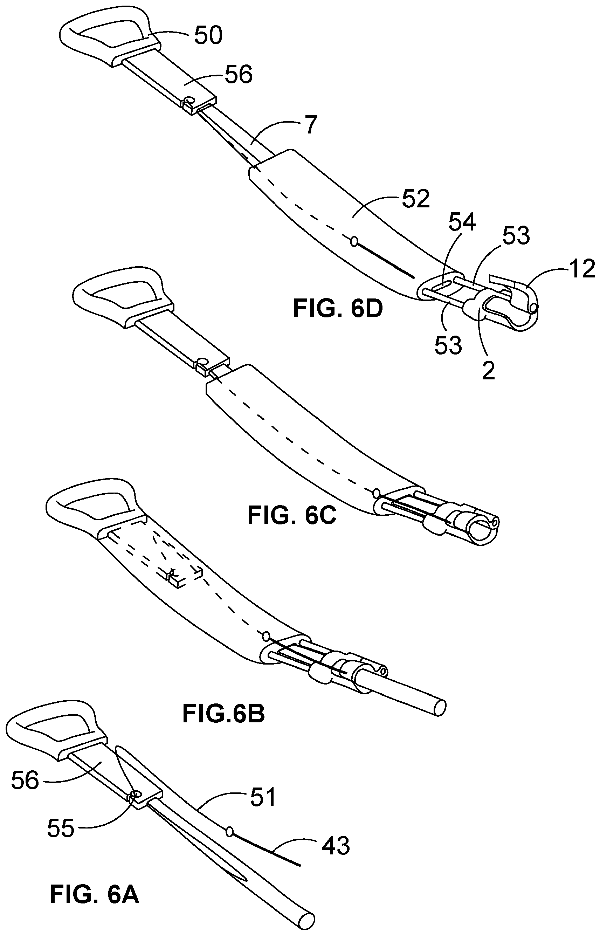

In this respect, Figures 5a to 5c and Figures 6a to 6d show an embodiment having a hinged cover 12, whereby the body can be opened to release the stent device, post unsheathing and once deployed. With this embodiment, deployment of the stent device by a single user is further facilitated.

In this connection, the body 2 takes the form of an opening clasp body 2 configured to hold, encase and support the stent device for insertion and unsheathing, which also facilitates removal from the device post unsheathing and deployment by the utilisation of a hinged cover element 12, namely a clasp lid which forms part of the body 2.

The body 2 provides a near circular encasement around the sheath split region and comprises a clasp housing 40, clasp lid 12, pivot 42 and locking pin 43.

The clasp body 2 is held closed through the locking pin 43 which is engaged within a pocket cavity 44 of the clasp lid. The engaged position is maintained throughout the deployment process up until the sheath has been fully removed from the stented section of the device.

The other end of the locking pin 43 is attached to the pull strap 50 through a link element 51, which in one embodiment may be a flexible resilient cord 51 of a specified length, which enables the pull strap to be engaged and pulled by the user to facilitate device deployment. The cord length is specifically configured to provide a controlled amount of slack to ensure that the pin is not in tension. The locking pin 43 remains engaged up until the sheath has been fully removed from the stented device section. In this respect the sheath material 7 is also coupled to the pull strap, so that the action of pulling the pull strap first applies tension to the sheath material at the distal end of the apparatus.

When the applied pull has taken up the pre-slack within the cord, this pull action is then transmitted to the locking pin, causing it to slide out from its engagement within the clasp lid pocket 44.

In another embodiment, the flexible cord element may be replaced with a resilient wire, which has an integral stop feature, that permits a pre-determined amount of sliding prior to engagement of the said stop feature.

Once the locking pin 43 is removed from the clasp lid 12, the lid is then free to pivot open to facilitate removal of the clasp body 2 from the deployed stent device 6.

Beneficially, the clasp body 2 fully supports the compacted compatible stent device 3 throughout the deployment process and that the lid 12 is only allowed to open towards the end of the sheath removal process, which can be performed in one continuous seamless movement without the need for a user to act on another deployment step.

As shown in Figures 6a to 6d, the pull strap 50 includes a handle 52 connected to the clasp body 2 by one or more connectors 53. The connectors 53 are preferably formed of flexible material allowing the handle to be manoeuvred so as to be angled with respect to the body, as shown in Figure 6b. This allows a user to manipulate the deployment apparatus in order to have better access for stent device deployment.

The handle 52 includes a through channel 54 through which the sheath material 7, locking pin 43 and cord 51 can pass as shown in Figure 6b. The cord from the pin 43 passes up the channel 54 and attaches to an aperture 55 in a key blade element 56 of the pull strap handle 50. The key blade element slots neatly away into the handle aperture prior to deployment of the stent device and withdrawal of the locking pin 43 from the cavity 44.

With the above apparatus, the body 2 holds and supports the compatible sheathed stent device 3 to enable the proximal compacted section to be inserted into either a native vessel or an adjunctive stent device body, so that it can be held for subsequent unsheathing and deployment to then enable vessel profusion to be reinstated.

This simplifies the delivery system in terms of its complexity, which together with the reduction in components provides the user with fewer procedural steps and potential risks, enabling a more time efficient and simplified device deployment.

The internal arrangement within the body enables controlled parting of the sheath when the user pulls the strap element. When the sheath is pulled across the internal bore restriction, the circular lumen aspect of the sheath is caused to continue to split, propagating along the two tail elements 7. Simultaneously, the movement applied at the strap is transmitted to the proximal end of the sheath, causing it to slide over the stent device, enabling the compacted stent device to be relieved from its radial constraint. In doing so, the stent device opens and engages the internal lumen of the vessel.

As shown in Figures 1 and 5, the compatible stent may have an integral stent device tip 24 feature. As shown in Figure 5, this may be provided at the proximal end of the stented region of the stent device, which when compacted within the sheath constraint also protrudes beyond the end of the sheath to partially expose said compacted stent device elements covered in soft suture (or PTFE thread) to provide an atraumatic tip like feature.

Whilst the restriction 5 is shown as an abutment within the bore 3, it may take the alternative suitable forms, such for example as pins or prong elements that temporarily inter-engage with formations such as eyelets or pockets attached to or formed on the stent device, to provide an alternative arrangement to hold and locate the compatible stent device within the body during unsheathing and deployment steps.

The body 2 may further have guide channels for the sheath to be drawn apart in a direction substantially perpendicular to the axis of the sheath, in so doing facilitating efficient low force splitting and removal of the sheath from the compacted device, while also providing a cavity location feature for accommodating additional device features (re-cuff element) adjacent to the stented section. This may also provide sufficient encasement of this fabric, or retention of it, to enable it to be utilized as a fabric backstop.

The collar 8 at the end of the body may further incorporate one or more rollers to facilitate the tails being pulled over a range of angles relative to the sheath axis.

## Claims

1. A stent device deployment apparatus (1) comprising:
a) a body (2) having an internal bore (4), and a distal end, a side window (10), and a longitudinal axis, the body including a hinged cover (12) and further including a locking pin (43) that locks the hinged cover (12) in a closed position, the body (2) further including a bore restriction (5) at the distal end of the body (2), the bore restriction including two circumferential slots extending along the longitudinal axis;
b) a sheath (7) extending through the internal bore (4) and the bore restriction (5); and
c) a pull strap (50) coupled to the sheath (7).

2. The apparatus (1) of claim 1, wherein the slots each subtend an angle in the range of 90 to 120 degrees circumferentially about the longitudinal axis.

3. The apparatus (1) of claim 1, further including an end collar at the distal end of the body (2).

4. The apparatus (1) of claim 3, wherein the body (2) includes inwardly facing walls that taper inwardly towards the end collar.

5. The apparatus (1) of claim 1, wherein the locking pin (43) is couplable to the pull strap.

6. The apparatus (1) of claim 5, further including a cord (51), wherein the locking pin (43) is coupled to the pull strap (50) by the cord (51), and wherein the cord (51) has a length of slack relative to the extent of sheath material coupled to the pull strap (50) to pull the locking pin (43) and thereby unlock the cover (12) after pulling the pull-strap (50) a sufficient distance to remove the sheath (7) from the body (2).

7. The apparatus (1) of claim 6, wherein the pull strap (50) includes a handle (52) that defines an aperture through which the sheath material at the distal end (25) of the body (2) extends.

8. The apparatus (1) of claim 7, wherein the handle (52) is coupled to the body (2) by way of one or more flexible members.

9. The apparatus (1) of claim 1, further including a profiled element at the bore restriction (5).

10. The apparatus (1) of claim 9, wherein the profiled element is a wedge member.

11. The apparatus (1) of claim 1, further including a stent device (3) collapsed in an un-deployed state, within the body (2).

## Patentansprüche

1. Eine Einrichtung (1) zum Entfalten einer Stentvorrichtung, die Folgendes beinhaltet:
a) einen Körper (2) mit einer Innenbohrung (4) und einem distalen Ende, einem Seitenfenster (10) und einer Längsachse, wobei der Körper eine schwenkbare Abdeckung (12) umfasst und ferner einen Verriegelungsstift (43) umfasst, der die schwenkbare Abdeckung (12) in einer geschlossenen Position verriegelt, wobei der Körper (2) ferner eine Bohrungsverengung (5) an dem distalen Ende des Körpers (2) umfasst, wobei die Bohrungsverengung zwei Umfangsschlitze umfasst, die sich entlang der Längsachse erstrecken;
b) eine Hülse (7), die sich durch die Innenbohrung (4) und die Bohrungsverengung (5) erstreckt; und
c) ein Zugband (50), das mit der Hülse (7) gekoppelt ist.

2. Einrichtung (1) gemäß Anspruch 1, wobei die Schlitze jeweils einem Winkel in dem Bereich von 90 bis 120 Grad in Umfangsrichtung um die Längsachse gegenüberliegen.

3. Einrichtung (1) gemäß Anspruch 1, die ferner einen Endkragen an dem distalen Ende des Körpers (2) umfasst.

4. Einrichtung (1) gemäß Anspruch 3, wobei der Körper (2) nach innen gerichtete Wände umfasst, die sich nach innen zu dem Endkragen hin verjüngen.

5. Einrichtung (1) gemäß Anspruch 1, wobei der Verriegelungsstift (43) mit dem Zugband koppelbar ist.

6. Einrichtung (1) gemäß Anspruch 5, die ferner eine Schnur (51) umfasst, wobei der Verriegelungsstift (43) durch die Schnur (51) mit dem Zugband (50) gekoppelt ist und wobei die Schnur (51) eine Länge an Spiel relativ zu dem Ausmaß des mit dem Zugband (50) gekoppelten Hülsematerials aufweist, um den Verriegelungsstift (43) zu ziehen und dadurch die Abdeckung (12) zu entriegeln, nachdem das Zugband (50) eine ausreichende Strecke gezogen wurde, um die Hülse (7) von dem Körper (2) zu entfernen.

7. Einrichtung (1) gemäß Anspruch 6, wobei das Zugband (50) einen Griff (52) umfasst, der eine Öffnung definiert, durch die sich das Hülsenmaterial an dem distalen Ende (25) des Körpers (2) erstreckt.

8. Einrichtung (1) gemäß Anspruch 7, wobei der Griff (52) mit dem Körper (2) mittels einem oder mehreren flexiblen Teilen gekoppelt ist.

9. Einrichtung (1) gemäß Anspruch 1, die ferner ein profiliertes Element an der Bohrungsverengung (5) umfasst.

10. Einrichtung (1) gemäß Anspruch 9, wobei das profilierte Element ein Keilteil ist.

11. Einrichtung (1) gemäß Anspruch 1, die ferner eine Stentvorrichtung (3) umfasst, die in einem nicht entfalteten Zustand innerhalb des Körpers (2) zusammengeklappt ist.

## Revendications

1. Un appareil (1) de déploiement de dispositif d'endoprothèse comprenant :
a) un corps (2) ayant un alésage interne (4), et une extrémité distale, une fenêtre latérale (10), et un axe longitudinal, le corps incluant un couvercle articulé (12) et incluant en outre une goupille de verrouillage (43) qui verrouille le couvercle articulé (12) dans une position fermée, le corps (2) incluant en outre une restriction d'alésage (5) au niveau de l'extrémité distale du corps (2), la restriction d'alésage incluant deux fentes circonférentielles qui s'étendent le long de l'axe longitudinal ;
b) une gaine (7) s'étendant à travers l'alésage interne (4) et la restriction d'alésage (5) ; et
c) une languette de traction (50) couplée à la gaine (7).

2. L'appareil (1) de la revendication 1, où les fentes sous-tendent chacune un angle compris dans un intervalle de 90 à 120 degrés circonférentiellement autour de l'axe longitudinal.

3. L'appareil (1) de la revendication 1, incluant en outre un collier d'extrémité au niveau de l'extrémité distale du corps (2).

4. L'appareil (1) de la revendication 3, où le corps (2) inclut des parois tournées vers l'intérieur qui s'inclinent vers l'intérieur en direction du collier d'extrémité.

5. L'appareil (1) de la revendication 1, où la goupille de verrouillage (43) peut être couplée à la languette de traction.

6. L'appareil (1) de la revendication 5, incluant en outre un cordon (51), où la goupille de verrouillage (43) est couplée à la languette de traction (50) par le cordon (51), et où le cordon (51) a une longueur de mou par rapport à l'étendue de matériau de gaine couplé à la languette de traction (50) pour tirer la goupille de verrouillage (43) et ainsi déverrouiller le couvercle (12) après avoir tiré la languette de traction (50) sur une distance suffisante pour retirer la gaine (7) du corps (2).

7. L'appareil (1) de la revendication 6, où la languette de traction (50) inclut une poignée (52) qui définit une ouverture à travers laquelle s'étend le matériau de gaine au niveau de l'extrémité distale (25) du corps (2).

8. L'appareil (1) de la revendication 7, où la poignée (52) est couplée au corps (2) au moyen d'un ou de plusieurs organes souples.

9. L'appareil (1) de la revendication 1, incluant en outre un élément profilé au niveau de la restriction d'alésage (5).

10. L'appareil (1) de la revendication 9, où l'élément profilé est un organe de calage.

11. L'appareil (1) de la revendication 1, incluant en outre un dispositif (3) d'endoprothèse rétracté dans un état non déployé, au sein du corps (2).
